(19) Europäisches Patentamt
European Patent Office
Office européen des brevets

(11) Veröffentlichungsnummer: **0 364 396 A1**

(12) # EUROPÄISCHE PATENTANMELDUNG

(21) Anmeldenummer: 89810688.5

(22) Anmeldetag: 14.09.89

(51) Int. Cl.5: **C07D 413/04 , C07D 417/04 , A01N 43/82 , //(C07D413/04, 271:00,271:00),(C07D417/04, 285:00,285:00),(C07D417/04, 285:00,271:00)**

Patentansprüche für folgenden Vertragsstaat: ES.

(30) Priorität: 23.09.88 CH 3537/88
24.02.89 CH 673/89

(43) Veröffentlichungstag der Anmeldung:
18.04.90 Patentblatt 90/16

(84) Benannte Vertragsstaaten:
AT BE CH DE ES FR GB GR IT LI LU NL

(71) Anmelder: **CIBA-GEIGY AG**
**Klybeckstrasse 141**
**CH-4002 Basel(CH)**

(72) Erfinder: **Beriger, Ernst, Dr.**
**Grabenmattweg 29**
**CH-4123 Allschwil(CH)**
Erfinder: **Eckhardt, Wolfgang, Dr.**
**Breslauerstrasse 14**
**D-7850 Lörrach(DE)**

(54) **Nematizide und fungizide Mittel.**

(57) Es werden Mercapto-bis-[1,3,4-oxa- und -thiadiazole]

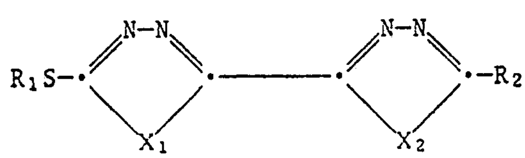 (I),

worin
$X_1$ und $X_2$ voneinander unabhängig Sauerstoff oder Schwefel,
$R_1$ Wasserstoff, unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,
$R_2$ Wasserstoff, $C_1$-$C_5$-Alkyl, unsubstituiertes oder durch Halogen substituiertes $C_1$-$C_3$-Alkoxy, unsubstituiertes oder durch Halogen substituiertes $C_1$-$C_3$-Alkylthio, unsubstituiertes oder durch Halogen substituiertes $C_3$-$C_7$-Alkenylthio, unsubstituiertes oder durch Halogen substituiertes $C_3$-$C_7$-Alkinylthio, -SH, unsubstituiertes oder durch Halogen substituiertes Phenyl oder

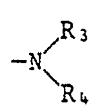

bedeuten, worin $R_3$ und $R_4$ für $C_1$-$C_3$-Alkyl stehen, sowie Verfahren zur Herstellung der Verbindungen der Formel I beschrieben.
Die Verbindungen der Formel I besitzen nematizide und mikrobizide Eigenschaften. Es werden nematizide

EP 0 364 396 A1

Mittel, die als Wirkstoff mindestens einen Wirkstoff der Formel I enthalten, ferner Verfahren zur Verwendung der Wirkstoffe und der Mittel bei der Bekämpfung von Nematoden und pflanzenschädigenden Pilzen beschrieben.

# Nematizide und fungizide Mittel

Die vorliegende Erfindung betrifft neue substituierte Mercapto-bis-[1,3,4-oxa- und -thiadiazole], deren Herstellung sowie nematizide und fungizide Mittel, die als Wirkstoff mindestens eine dieser Verbindungen enthalten. Die Erfindung betrifft ferner Verfahren zur Herstellung der Wirkstoffe, ihre Verwendung und Mittel zur Bekämpfung von Nematoden, insbesondere von pflanzenschädigenden Nematoden, sowie Pilzen, insbesondere von pflanzenschädigenden bodenbürtigen Pilzen.

Die erfindungsgemässen Mercapto-bis-[1,3,4-oxa- und -thiadiazole] entsprechen der allgemeinen Formel I

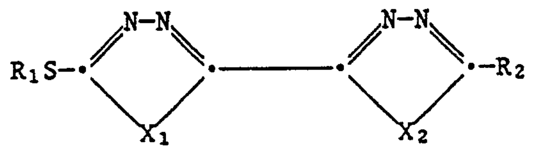

(I),

worin
$X_1$ und $X_2$ voneinander unabhängig Sauerstoff oder Schwefel,
$R_1$ Wasserstoff, unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,
$R_2$ Wasserstoff, $C_1$-$C_5$-Alkyl, unsubstituiertes oder durch Halogen substituiertes $C_1$-$C_3$-Alkoxy, unsubstituiertes oder durch Halogen substituiertes $C_1$-$C_3$-Alkylthio, unsubstituiertes oder durch Halogen substituiertes $C_3$-$C_7$-Alkenylthio, unsubstituiertes oder durch Halogen substituiertes $C_3$-$C_7$-Alkinylthio, -SH, unsubstituiertes oder durch Halogen substituiertes Phenyl oder

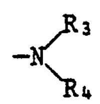

bedeuten, worin $R_3$ und $R_4$ für $C_1$-$C_3$-Alkyl stehen
sowie Salze dieser Verbindungen.

Unter Alkyl sind als selbständiger Rest sowie als Teil einer anderen Gruppe, wie Alkoxy, gerad- und verzweigtkettige Alkylgruppen zu verstehen. Dazu zählen die Methyl-, die Ethyl- sowie die normalen und isomeren Propyl-, Butyl- und Pentylgruppen. Halogensubstituiertes Alkyl steht für einen einfach bis perhalogenierten Alkylrest, wie z.B. $CHCl_2$, $CH_2F$, $CCl_3$, $CH_2Cl$, $CHFCH_3$, $CH_2CH_2Br$, $CF_2CF_3$, $C_2Cl_5$, $CH_2Br$, $CHBrCl$ usw., vorzugsweise für $CHF_2$. Für Alkylthio kann Methylthio, für Halogenalkylthio kann Difluormethylthio als Beispiel genannt werden, Alkenyl bedeutet z.B. Propenyl-(1), Allyl, Butenyl-(1), Butenyl-(2) oder Butenyl-(3) sowie Ketten mit mehreren Doppelbindungen. Halogenalkenyl bedeutet beispielsweise 3,4,4-Trifluor3-buten-1-yl. Als Halogenalkenylthio-Gruppe kann 3,4,4-Trifluor-3-buten-1-ylthio, als halogensubstituiertes Phenyl kann 4-Chlorphenyl genannt werden. Alkinyl bedeutet z.B. Propinyl-(2), Butinyl-(1), Butinyl-(2), Pentinyl-(4) usw.. Halogen steht für Fluor, Chlor, Brom oder Jod, vorzugsweise für Fluor, Chlor oder Brom. Als Dialkylamino Reste können beispielsweise Diethyl-, Diisopropyl-, vorzugsweise Dimethylamino genannt werden.

Beispiele salzbildender Säuren sind unter den anorganischen Säuren Halogenwasserstoffsäuren wie Fluorwasserstoffsäure, Chlorwasserstoffsäure, Bromwasserstoffsäure oder Jodwasserstoffsäure sowie ferner Schwefelsäure, Phosphorsäure, phosphorige Säure, Salpetersäure und unter den organischen Säuren Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Es sind bereits Oxadiazol- und Thiadiazol-Derivate bekannt, die als nematizid wirksam beschrieben sind. So sind in der US-Patentschrift 3,770,754 solche Verbindungen mit einer 1,2,4-Stellung der Heteroatome offenbart, während in der US-Patentschrift 4,454,147 1,3,4-Thiadiazol-Derivate beschrieben sind, bei denen im Vergleich mit den erfindungsgemässen Verbindungen der Heterocyclus anstelle der Mercapto-Gruppen durch ein Chloratom substituiert ist. Diese bekannten Verbindungen haben bisher als Nematizide die in der Praxis an sie gestellten Ansprüche nicht in vollem Umfang befriedigen können. Ferner sind Oxadiazol-Derivate mit fungizider Wirksamkeit in der DE-OS 2 361 613 beschrieben. Es werden darin jedoch keine dieser Verbindungen expressis verbis genannt, die unter den Umfang der erfindungsgemässen Formel I fallen.

Durch die Bereitstellung der erfindungsgemässen Verbindungen der Formel I ist es nun gelungen einen wertvollen Beitrag zur Bekämpfung der beträchtliche landwirtschaftliche Schäden an Pflanzgut verursachenden Pflanzennematoden zu leisten. Auf diese Weise können Ernteeinbussen bei Kulturpflanzen wie z.B. Kartoffeln, Getreide, Rüben, Raps, Kohl, Tabak, Sojabohnen, Baumwolle und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Wirkstoffen die Nematodengattungen Ditylenchus (Stengelparasiten), Aphelenchoides (Blattnematoden) und Anguina (Blütennematoden) wirkungsvoll bekämpfen.

Mit den Wirkstoffen der Formel I lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) und ferner der Gattung Globodera, wie z.B. Globodera rostochiensis (Kartoffelzystennematode) sowie Vertreter von wandernden Endoparasiten, wie z.B. Pratylenchus penetrans oder Radopholus similis und Vertreter von Ektoparasiten, wie z.B. Trichodorus spp. und Xiphinema spp. erfolgreich bekämpfen.

Ebenso lassen sich vorzugsweise mit den Wirkstoffen der Formel I besonders schädliche Bodenpilze der Gattung Pythium, wie z.B. Pythium ultimum, der Gattung Rhizoctonia, wie z.B. Rhizoctonia solani, sowie der Gattung Fungi imperfecti, wie z.B. Cercospora, Basidomyceten wie beispielsweise Puccinia, Ascomyceten, wie beispielsweise Erysiphe sowie Oomyceten, wie beispielsweise Phytophthora oder Plasmopara erfolgreich bekämpfen.

Zur Bekämpfung der Pflanzennematoden und Bodenpilze und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematoden- und Bodenpilz-Species und werden somit den Erfordernissen der Praxis gerecht. Die nematizide und fungizide Wirkungsweise der erfindungsgemässen Verbindungen wird durch eine geringe Phytotoxizität in vorteilhafter Weise begleitet, womit der allgemein wünschenswerten Verminderung der Umweltbelastung in besonderem Masse Rechnung getragen wird.

Eine nematizide Wirksamkeit wird durch Hemmung der Wurzelgallbildung an der behandelten Kulturpflanze im Vergleich zur unbehandelten Pflanze festgestellt und auch bestimmt.

Die Wirkung wird als "gut" bezeichnet, wenn der Befall der behandelten Pflanze geringer ist als 20 % des Befalls der unbehandelten Pflanze.

Im Rahmen der vorliegenden Erfindung sind als nematizide Wirkstoffe jene Verbindungen der Formel I bevorzugt, bei welchen $R_2$ Wasserstoff, $C_1$-$C_5$-Alkyl, Alkylthio, Difluormethylthio, Alkenylthio, 3,4,4-Trifluor-3-buten-1-ylthio, SH, Phenyl oder

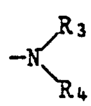

bedeutet.

Von dieser Gruppe sind jene Verbindungen bevorzugt, bei welchen $R_1$ Methyl, Difluormethyl, Trifluormethyl, Cyanomethyl, Allyl, Propargyl oder 3,4,4-Trifluor-3-buten-1-yl und $R_2$ Wasserstoff, Methyl, Difluormethylthio, 3,4,4-Trifluor-3-buten-1-ylthio, SH, Phenyl oder Dimethylamino bedeuten.

Von dieser Gruppe werden jene insbesondere bevorzugt, bei welchen $R_1$ Difluormethyl oder 3,4,4-Trifluorbuten-3-1-yl bedeutet.

Als Einzelverbindungen sind das 2-Difluormethylthio-5-(2-methyl-1,3,4-oxadiazol-5-yl)-1,3,4-oxadiazol, das 2-(3,4,4-Trifluor-3-buten-1-ylthio)-5-(2-methyl-1,3,4-oxadiazol-5-yl)-1,3,4-oxadiazol, das 2-Difluormethylthio-5-(2-methyl-1,3,4-oxadiazol-5-yl)-1,3,4-thiadiazol, das 2-(3,4,4-Trifluor-3-buten-1-ylthio)-5-(2-methyl-1,3,4-oxadiazol-5-yl)-1,3,4-thia diazol, das 2-(3,4,4-Trifluor-3-buten-1-ylthio)-5-(2-methylthio-1,3,4-thiadiazol-5-yl)-1,3,4-thiadiazol und das 2-(3,4,4-Trifluor-3-buten-1-ylthio)-5-(2-methylthio-1,3,4-thiadiazol-5-yl)-1,3,4-oxadiazol, 2-(Difluormethylthio)-5-(2-methyl-1,3,4-oxadiazol-5-yl)-1,3,4-thiadiazol, 2-(3,4,4-Trifluor-3-buten-1-ylthio)-5-(2-methyl-1,3,4-oxadiazol-5-yl)-1,3,4-thiadiazol, 2-(3,4,4-Trifluor-3-buten-1-ylthio)-5-(2-mercapto-1,3,4-oxadiazol-5-yl)-1,3,4-thiadiazol, 2-(3,4,4-Trifluor-3-buten-1-ylthio)-5-[2-(3,4,4-trifluor-3-buten-1-ylthio)-1,3,4-oxadiazol-5-yl]-1,3,4-thiadiazol, 2-(3,4,4-Trifluor-3-buten-1-ylthio)-5-(2-phenyl-1,3,4-oxadiazol-5-yl)-1,3,4-thiadiazol, 2-(3,4,4-Trifluor-3-buten-1-ylthio)-5-(2-phenyl-1,3,4-oxadiazol-5-yl)-1,3,4-oxadiazol, 2-(3,4,4-Trifluor-3-buten-1-ylthio)-5-(2-difluormethylthio-1,3,4-oxadiazol-5-yl)-1,3,4-thiadiazol, 2-(3,4,4-Trifluor-3-buten-1-ylthio)-5-[2-(3,4,4-trifluor-3-buten-1-ylthio)-1,3,4-oxadiazol-5-yl]-1,3,4-oxadiazol, hervorzuheben.

Die Verbindungen der Formel I werden erfindungsgemäss hergestellt, indem man

a) in einer Kondensationsreaktion eine Verbindung der Formel II

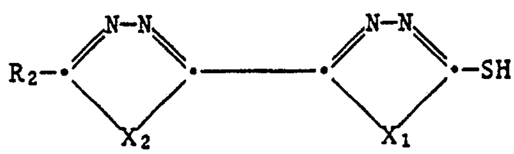

(II),

oder eine Verbindung der Formel III

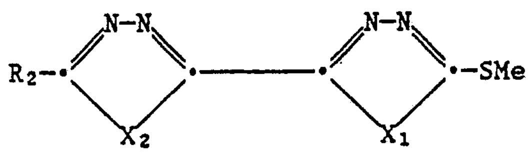

(III),

mit einer Verbindung der Formel IV

Hal-$R_1$ (IV)

in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

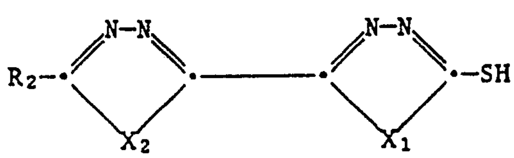

(II),

mit einer Verbindung der Formel V

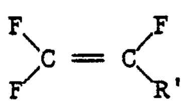

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ie

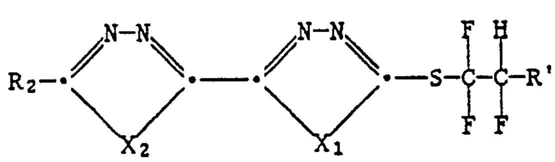

(Ie)

oder zu einer Verbindung der Formel If

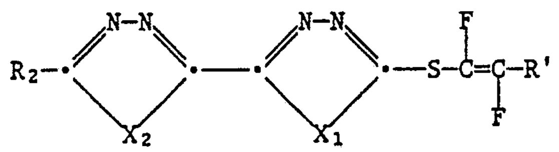

(If)

führt, wobei in den vorstehend genannten Formeln II, III, Ie, If, IV und V Me für ein Alkalimetall oder für Ammonium steht, Hal Halogen, vorzugsweise Chlor, Brom oder Jod bedeutet, und R′ Fluor oder Trifluormethyl darstellt, während $R_1$ und $R_2$ die unter Formel I angegebenen Bedeutungen besitzen.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon sowie Wasser und Alkohole wie z.B. Methanol, Ethanol, Isopropanol oder Butanol; und ganz allgemein Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.butylat oder Natriummethylat.

Die Zugabe katalytischer Mengen eines Kronenethers, wie z.B. 18-Krone-6 oder 15-Krone-5, wirkt sich in den Herstellungsverfahren günstig auf den Reaktionsablauf aus. Ferner hat sich für den gleichen Zweck die katalytische Verwendung von Tetraalkylaminsalzen, z.B. Tetraalkylammoniumchloride oder -bromide, vorzugsweise Tetra-n-butylammoniumbromid, als vorteilhaft erwiesen. Darüber hinaus sind Alkalijodide, vorzugsweise Kaliumjodid, als Katalysatoren vorteilhaft einsetzbar.

In den Herstellungsverfahren betragen die Reaktionstemperaturen 10 bis 90 °C, vorzugsweise 30° bis 80 °C. Für die Druckverhältnisse während des Reaktionsablaufs sind 1 bis 20 bar, vorzugsweise 6 bis 14 bar, massgebend.

Die Ausgangsverbindungen der Formel II sind teils bekannt, teils neu. Die neuen Verbindungen der Formel IIa

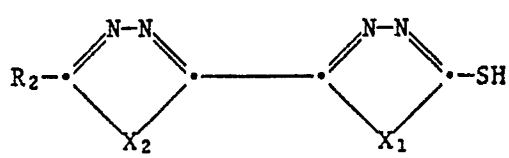

(IIa),

worin $R_2$, $X_1$ und $X_2$ die unter Formel I angegebene Bedeutungen haben, mit der Massgabe, dass $R_2$ eine von Wasserstoff verschiedene Bedeutung hat, wenn $X_1$ und $X_2$ Schwefel bedeuten, sind Zwischenprodukte zur Herstellung wertvoller nematozider Wirkstoffe und bilden damit einen Bestandteil der vorliegenden Erfindung.

Hervorzuheben sind dabei die Verbindungen der Formel IIa, worin $R_2$, $X_1$ und $X_2$ die unter Formel I angegebene Bedeutung haben, mit der Massgabe, dass $X_1$ und $X_2$ nicht gleichzeitig Schwefel bedeuten.

Die Ausgangsverbindungen der Formel II können nach bekannten Methoden wie folgt hergestellt werden:

a) Die 2-Mercapto-1,3,4-oxadiazole sind gewinnbar durch Zugabe von Schwefelkohlenstoff zu einer Lösung des entsprechend substituierten Hydrazids in alkoholisch-wässrigem Kaliumhydroxid und Erhitzen des Reaktionsgemisches während einiger Stunden. Als Lösungsmittel werden dabei Alkohole wie z.B. Ethylalkohol oder n-Amylalkohol verwendet. Durch Ansäuern der gebildeten Kaliumsalze werden die freien Mercapto-Verbindungen erhalten [vgl. J.Am.Chem.Soc. 78, 4975-4978 (1956)]. Die 2-Mercapto-1,3,4-oxadiazole sind ferner gewinnbar durch Umsetzung des entsprechenden Acylhydrazids mit Thiophosgen in einem inerten Lösungsmittel wie z.B. Dioxan [vgl. J.Org.Chem. 26, 88-95 (1961)].

b) Die 2-Mercapto-1,3,4-thiadiazole sind erhältlich durch Behandlung des entsprechend substituierten

Acyl-kaliumdithiocarbazats mit konzentrierter Schwefelsäure bei -5° bis 10° C [vgl. J.prakt.Chem. 93, 49 (1916); J.Org.Chem. 23, 1021 (1958); J.Heterocycl.Chem. 19, 542-544 (1982)].

Die Erfindung betrifft auch Mittel, zur Bekämpfung von pflanzenschädigenden Nematoden und Pilzen sowie zur präventiven Verhütung des Nematoden- und Pilzbefalls von Pflanzgut, welche die Wirkstoffe der Formel I enthalten.

Darüber hinaus schliesst die vorliegende Erfindung zusätzlich die Herstellung nematizider und fungizider Mittel ein, die gekennzeichnet ist durch das innige Vermischen von Aktivsubstanzen der Formel I mit einer oder mehreren hierin beschriebenen Trägerstoffen und Hilfsingredienzen. Eingeschlossen ist auch ein Verfahren zur Behandlung von Pflanzen, das durch die Applikation der Verbindungen der Formel I oder der neuen Mittel charakterisiert ist.

Ein bezvorzugtes Verfahren für den Einsatz eines Wirkstoffs der Formel I bzw. eines nematiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produkticnssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugsweise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhält nissen entsprechend gewählt. Günstige Aufwandmengen liegen im allgemeinen bei 500 g bis 6 kg Aktivsubstanz (AS) je ha; bevorzugt 1 bis 4 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien genannt die Alkali-, Erdalkali- oder gegebenenfalls substituierten Ammoniumsalze von

höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze sowie modifizierte und nicht modifizierte Phospholipide zu erwähnen.

Häufiger werden jedoch sogenannte synthetische Tenside verwendet, insbesondere Fettalkoholsulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise zwei Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

1. Herstellungsbeispiele

Beispiel H1 2-Mercapto-5-(2-methyl-1,3,4-oxadiazol-5-yl)-1,3,4-oxadiazol (Verbindung Nr. 1.5)

3,6 g 5-Methyl-1,3,4-oxadiazol-2-carbonsäurehydrazid vom Schmelzpunkt 150-151 °C, auf übliche Art

aus dem entsprechenden Aethylester [Canad.J. Chem. 65, 166 (1987)] hergestellt, werden in 100 ml Dioxan mit 2,9 g Thiophosgen über Nacht bei Raumtemperatur und anschliessend während 5 Stunden bei 55° C verrührt. Nach dem Erkalten filtriert man ungelöste Feststoffe ab, gibt zum Filtrat 200 ml hexan, wobei das gewünschte Produkt (4 g mit Zersetzungspunkt 225°) auskristallisiert.

Beispiel H2 2-Mercapto-5-(2-dimethylamino-1,3,4-oxadiazol-5-yl)-1,3,4-oxadiazol (Verbindung Nr. 1.6)

8,5 g 5-Dimethylamino-oxadiazol-2-carbonsäurehydrazid vom Schmelzpunkt 151-153° C, auf übliche Art aus dem entsprechenden Aethylester (J.Heterocyclic Chem. 14, (1977) 1385) hergestellt, werden in 200 ml Dioxan mit 5,7 g Thiophosgen über Nacht bei Raumtemperatur und anschliessend noch während 4 Stunden bei 70° verrührt. Nach Abkühlen der Reaktionsmischung auf 20° werden die Festteile abgenutscht und mit Wasser verrührt. Nach dem Trocknen der Kristalle erhält man die Titelverbindung (8,3 g) vom Schmelzpunkt 227-29° C (Zersetzung).

Beispiel H3 2-(3,4,4-Trifluor-3-buten-1-ylthio)-5-(2-methyl-1,3,4-oxadiazol-5-yl)-1,3,4-oxadiazol (Verbindung Nr. 2.28)

3,7 g 2-Mercapto-5-(2-methyl-1,3,4-oxadiazol-5-yl)-1,3,4-oxadiazol gemäss Beispiel H1 werden in einer Mischung von 36 ml Dioxan und 4 ml Dimethylformamid vorgelegt und unter Rühren mit 2,24 g Kalium-t-butylat versetzt. Man lässt 4,2 g 4-Brom-1,1,2-trifluor-buten-1 zutropfen und rührt die Mischung über Nacht bei Raumtemperatur. Nach dem Abfiltrieren der Salze destilliert man die Lösungsmittel im Vakuum ab, nimmt den Rückstand in Methylenchlorid auf und wäscht die Lösung nacheinander mit Wasser und 1-normaler Natronlauge aus und verdampft das Lösungsmittel im Vakuum. Als Rückstand erhält man 3,2 g der gewünschten Verbindung mit Schmelzpunkt 77-79° C.

Beispiel H4 2-(3,4,4-Trifluor-3-buten-1-ylthio)-5-(2-dimethylamino-1,3,4-oxadiazol-5-yl)-1,3,4-oxadiazol (Verbindung Nr. 2.32)

4,26 g 2-Mercapto-5-(2-dimethylamino-1,3,4-oxadiazol-5-yl)-1,3,4-oxadiazol gemäss Beispiel H2 werden in 40 ml Dioxan vorgelegt und mit 2,24 g Kalium-t-butylat verrührt. Man gibt 4 ml Dimethylformamid und anschliessend 4,2 g 4-Brom-1,1,2-trifluorbuten-1 zu und rührt die Mischung über Nacht bei Raumtemperatur. Man giesst die Mischung auf Wasser und nutscht die Kristalle ab. Nach Trocknen und Verrühren mit Aether erhält man 4,6 g der Titelverbindung mit Schmelzpunkt 87-90° C.

Analog der vorstehenden Herstellungsbeispiele sowie der vorbeschriebenen Verfahren lassen sich folgende Zwischenprodukte und erfindungsgemässe Verbindungen herstellen. Die nachfolgend aufgeführten Verbindungen dienen der Illustration der vorliegenden Erfindung und stellen keine Begrenzung derselben dar.

Tabelle 1 (Zwischenprodukte)

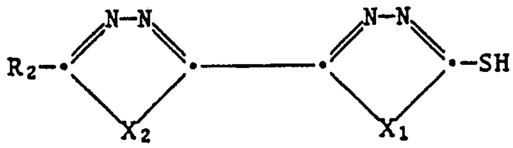

(II)

| Verb. Nr. | $R_2$ | $X_2$ | $X_1$ | Physik. Daten |
|---|---|---|---|---|
| 1.1 | H | O | O | |
| 1.2 | H | O | S | |
| 1.3 | H | S | O | |
| 1.4 | H | S | S | |
| 1.5 | $CH_3$ | O | O | Smp.: 225-227°C |
| 1.6 | $(CH_3)_2N$ | O | O | Smp.: 227-229°C |
| 1.7 | $CH_3S$ | S | S | Smp.: 179-182°C |
| 1.8 | $CH_3S$ | S | O | Smp.: 190-194°C |
| 1.9 | $CH_3O$ | S | O | |
| 1.10 | $CH_3O$ | S | S | |
| 1.11 | $CHF_2S$ | S | O | |
| 1.12 | $CHF_2S$ | S | S | |
| 1.13 | SH | O | O | Smp.: 236-238°C |
| 1.14 | $CH_3$ | O | S | Smp.: 204-207°C |

Tabelle 2

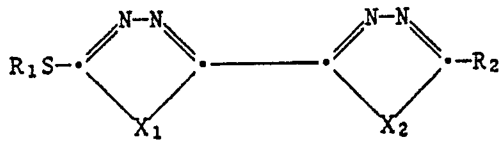

(I)

| Verb. Nr. | $R_1$ | $R_2$ | $X_1$ | $X_2$ | Physik. Daten |
|---|---|---|---|---|---|
| 2.1 | $CH_3$ | H | O | O | |
| 2.2 | $CH_2CN$ | H | O | O | |
| 2.3 | $CH_2CN$ | H | O | S | |
| 2.4 | $CH_2CH=CH_2$ | H | O | O | |
| 2.5 | $CH_2CH=CH_2$ | H | O | S | |
| 2.6 | $CH_2-C\equiv CH$ | H | O | O | |
| 2.7 | $CH_2-C\equiv CH$ | H | O | S | |
| 2.8 | $CH_2-C\equiv CH$ | H | S | O | |
| 2.9 | $CH_2-C\equiv CH$ | H | S | S | |
| 2.10 | $CF_3$ | H | O | O | |
| 2.11 | $CF_3$ | H | O | S | |
| 2.12 | $CHF_2$ | H | O | O | |
| 2.13 | $CHF_2$ | H | O | S | |
| 2.14 | $CHF_2$ | H | S | O | |
| 2.15 | $CHF_2$ | H | S | S | |
| 2.16 | $CHF_2$ | $CH_3$ | O | O | |
| 2.17 | $CHF_2$ | $CH_3$ | O | S | |
| 2.18 | $CHF_2$ | $CH_3$ | S | O | Smp.: 103-106°C |
| 2.19 | $CHF_2$ | $CH_3$ | S | S | |
| 2.20 | $CHF_2$ | $(CH_3)_2N$ | O | O | |
| 2.21 | $CHF_2$ | $(CH_3)_2N$ | O | S | |
| 2.22 | $CHF_2$ | $(CH_3)_2N$ | S | O | |
| 2.23 | $CHF_2$ | $(CH_3)_2N$ | S | S | |
| 2.24 | $CH_2-CH_2-CF=CF_2$ | H | O | O | |
| 2.25 | $CH_2-CH_2-CF=CF_2$ | H | O | S | |
| 2.26 | $CH_2-CH_2-CF=CF_2$ | H | S | O | |
| 2.27 | $CH_2-CH_2-CF=CF_2$ | H | S | S | |
| 2.28 | $CH_2-CH_2-CF=CF_2$ | $CH_3$ | O | O | Smp.: 77-79°C |
| 2.29 | $CH_2-CH_2-CF=CF_2$ | $CH_3$ | O | S | |
| 2.30 | $CH_2-CH_2-CF=CF_2$ | $CH_3$ | S | O | Smp.: 69-71°C |
| 2.31 | $CH_2-CH_2-CF=CF_2$ | $CH_3$ | S | S | |
| 2.32 | $CH_2-CH_2-CF=CF_2$ | $(CH_3)_2N$ | O | O | Smp.: 87-90°C |
| 2.33 | $CH_2-CH_2-CF=CF_2$ | $(CH_3)_2N$ | O | S | |
| 2.34 | $CH_2-CH_2-CF=CF_2$ | $(CH_3)_2N$ | S | O | |
| 2.35 | $CH_2-CH_2-CF=CF_2$ | $(CH_3)_2N$ | S | S | |
| 2.36 | $CHF_2$ | $CH_3S$ | O | S | Smp.: 68-70°C |
| 2.37 | $CHF_2$ | $CH_3S$ | S | S | Smp.: 100-102°C |
| 2.38 | $CH_2-CH_2-CF=CF_2$ | $CH_3S$ | O | S | Smp.: 68-70°C |
| 2.39 | $CH_2-CH_2-CF=CF_2$ | $CH_3S$ | S | S | Smp.: 93-97°C |
| 2.40 | $CHF_2$ | $CH_3O$ | O | S | |

Tabelle 2 (Fortsetzung)

| Verb. Nr. | $R_1$ | $R_2$ | $X_1$ | $X_2$ | Physik. Daten |
|---|---|---|---|---|---|
| 2.41 | $CHF_2$ | $CH_3O$ | S | S | |
| 2.42 | $CH_2-CH_2-CF=CF_2$ | $CH_3O$ | O | S | |
| 2.43 | $CH_2-CH_2-CF=CF_2$ | $CH_3O$ | S | S | |
| 2.44 | $CHF_2$ | $CHF_2S$ | O | S | |
| 2.45 | $CHF_2$ | $CHF_2S$ | S | S | |
| 2.46 | $CH_2-CH_2-CF=CF_2$ | $CHF_2S$ | O | S | |
| 2.47 | $CH_2-CH_2-CF=CF_2$ | $CHF_2S$ | S | S | |
| 2.48 | $CH_2-CH_2-CF=CF_2$ | SH | S | O | Smp. 132°C (Zers.) |
| 2.49 | $CH_2-CH_2-CF=CF_2$ | $SCH_2-CH_2-CF=CF_2$ | S | O | Oel |
| 2.50 | $CH_2-CH_2-CF=CF_2$ | $C_6H_5$ | S | O | Smp. 90-92°C |
| 2.51 | H | $C_6H_5$ | S | O | Smp. 177-185°C |
| 2.52 | H | $C_6H_5$ | O | O | Smp. 195°C (Zers.) |
| 2.53 | $CH_2-CH_2-CF=CF_2$ | $C_6H_5$ | O | O | Harz |
| 2.54 | $CHF_2$ | $C_6H_5$ | S | O | Smp. 132-134°C |
| 2.55 | $CH_2-CH_2-CF=CF_2$ | $CHF_2S$ | S | O | Oel |
| 2.56 | $CH_2-CH_2-CF=CF_2$ | SH | S | S | |
| 2.57 | $CH_2-CH_2-CF=CF_2$ | $CHF_2S$ | S | S | |
| 2.58 | $CH_2-CH_2-CF=CF_2$ | $SCH_2-CH_2-CF=CF_2$ | S | S | |
| 2.59 | $CH_2-CH_2-CF=CF_2$ | $SCH_2-CH_2-CF=CF_2$ | O | O | $n_D^{22}$ 1,5339 |

Formulierungsbeispiele

F.2. Formulierungsbeispiele für flüssige Wirkstoffe der Formel I (% = Gewichtsprozent)

| F.2.1 Emulsions-Konzentrate | a) | b) | c) |
|---|---|---|---|
| Wirkstoff aus Tabelle 2 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| F.2.2 Lösungen | a) | b) | c) | d) |
|---|---|---|---|---|
| Wirkstoff aus Tabelle 2 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |
| (MG = Molekulargewicht) | | | | |

Die Lösungen sind zur Anwendung in Form kleinster Tropfen geeignet.

| F.2.3 Granulate | a) | b) |
|---|---|---|
| Wirkstoff aus Tabelle 2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F.2.4. Stäubemittel | a) | b) |
|---|---|---|
| Wirkstoff aus Tabelle 2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

F.3 Formulierungsbeispiele für feste Wirkstoffe der Formel I (% = Gewichtsprozent)

| F.3.1 Spritzpulver | a) | b) | c) |
|---|---|---|---|
| Wirkstoff aus Tabelle 2 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F.3.2 Emulsions-Konzentrat | |
|---|---|
| Wirkstoff aus Tabelle 2 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F.3.3 Stäubemittel | a) | b) |
|---|---|---|
| Wirkstoff aus Tabelle 2 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| F.3.4 Extruder Granulat | |
|---|---|
| Wirkstoff aus Tabelle 2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| F.3.5 Umhüllungs-Granulat | |
|---|---|
| Wirkstoff aus Tabelle 2 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| F.3.6 Suspensions-Konzentrat | |
|---|---|
| Wirkstoff aus Tabelle 2 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

B.1 Wirkung gegen Meloidogyne incognita auf Tomaten

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26±1 °C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Verbindungen aus Tabelle 2 zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmen z.B. die Verbindungen Nr. 2.28, 2.37, 2.38 und 2.39 im obigen Versuch die Wurzelgallbildung fast vollständig (0-10 % Restbefall).

B.2 Wirkung gegen Cercospora arachidicola auf Erdnusspflanzen Residual-protektive Wirkung

10-15 cm hohe Erdnusspflanzen werden mit einer aus Spritzpulver der Wirksubstanz hergestellten Spritzbrühe (200 ppM Aktivsubstanz) besprüht und 48 Stunden später mit einer Konidiensuspension des Pilzes infiziert. Die infizierten Pflanzen werden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigen Erdnusspflanzen, die mit Wirkstoffen aus der Tabelle behandelt werden, einen stark reduzierten Cercospora-Befall. Die Verbindung 2.38 verhindert das Auftreten von Flecken fast vollständig (0-10 %).

B.3 Wirkung gegen Plasmopara viticola auf Reben

a) Residual-protektive Wirkung

Im 4-5 Blattstadium wurden Rebensämlinge mit einer aus Spritzpulver des Wirkstoffes hergestellten Spritzbrühe (200 ppM Aktivsubstanz) besprüht. Nach 24 Stunden wurden die behandelten Pflanzen mit einer Sporangien suspension des Pilzes infiziert. Nach einer Inkubation während 6 Tagen bei 95-100 % relativer Luftfeuchtigkeit und 20 °C wurde der Pilzbefall beurteilt.

b) Residual-kurative Wirkung

Im 4-5 Blattstadium wurden Rebensämlinge mit einer Sporangiensuspension des Pilzes infiziert. Nach einer Inkubation während 24 Stunden in einer Feuchtkammer bei 95-100 % relativer Luftfeuchtigkeit und 20 °C wurden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 6 Tage nach der Infektion.

Verbindungen aus den Tabellen 1 und 2 zeigten gegen Plasmopara viticola auf Reben eine sehr gute fungizide Wirkung, insbesondere der Wirkstoff Nr. 2.37 bewirkte eine vollständige Unterdrückung des Pilzbefalls (Restbefall 0 bis 5 %).

B.4 Wirkung gegen Rhizoctonia solani (Bodenpilz auf Reispflanzen)

Protektiv-lokale Bodenapplikation

12 Tage alte Reispflanzen werden mit einer aus einer Formulierung des Wirkstoffes hergestellten Spritzbrühe (0,002 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27 °C (Tag), bzw. 23 °C (Nacht) und 100 % rel. Luftfeuchtigkeit (Feuchtkasten) in der Klimakammer wird der Pilzbefall auf Blattscheide, Blättern und Stengel beurteilt.

Verbindungen aus den Tabellen 1-2 zeigen gegen Rhizoctonia solani gute Aktivität. So zeigt die Verbindung 2.37 eine Wirkung von ca. 80 %.

B.5 Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

Verbindungen aus den Tabellen 1-2 zeigten gegen den Phytophthora-Pilz eine gute Schutzwirkung. So reduzierten z.B. die Verbindung 2.37 den Pilzbefall auf 0 bis 20 %. Unbehandelte jedoch infizierte Kontrollpflanzen wiesen dagegen einen 100 %igen Phytophthora-Befall auf.

B.6 Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22° C aufgestellt und der Pilzbefall nach 10 Tagen beurteilt.

Verbindungen aus den Tabellen 1-2 zeigten eine gute Wirkung gegen Erysiphe-Pilze. Unbehandelte, aber infizierte Kontrollpflanzen zeigten einen Erysiphe-Befall von 100 %. Unter anderen Verbindungen aus den Tabellen hemmten die Verbindung Nr. 2.36 den Pilzbefall bei Gerste auf 0-5 %.

## Ansprüche

1. Mercapto-bis-(1,3,4-oxa- und -thiadiazole) der Formel I

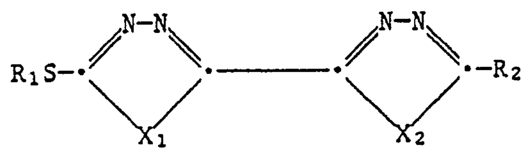

(I),

worin
$X_1$ und $X_2$ voneinander unabhängig Sauerstoff oder Schwefel,
$R_1$ Wasserstoff, unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,
$R_2$ Wasserstoff, $C_1$-$C_5$-Alkyl, unsubstituiertes oder durch Halogen substituiertes $C_1$-$C_3$-Alkoxy, unsubstituiertes oder durch Halogen substituiertes $C_1$-$C_3$-Alkylthio, unsubstituiertes oder durch Halogen substituiertes $C_3$-$C_7$-Alkenylthio, unsubstituiertes oder durch Halogen substituiertes $C_3$-$C_7$-Alkinylthio, -SH, unsubstituiertes oder durch Halogen substituiertes Phenyl oder

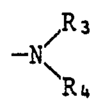

bedeuten, worin $R_3$ und $R_4$ für $C_1$-$C_3$-Alkyl stehen
sowie Salze dieser Verbindungen.

2. Mercapto-bis-(1,3,4-oxa- und -thiadiazole) gemäss Anspruch 1, worin
$R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,
$R_2$ Wasserstoff, $C_1$-$C_5$-Alkyl, unsubstituiertes oder durch Halogen substituiertes $C_1$-$C_3$-Alkoxy, unsubstituiertes oder durch Halogen substituiertes $C_1$-$C_3$-Alkylthio, unsubstituiertes oder durch Halogen substituiertes $C_3$-$C_7$-Alkenylthio, unsubstituiertes oder durch Halogen substituiertes $C_3$-$C_7$-Alkinylthio, -SH, unsubstituiertes oder durch Halogen substituiertes Phenyl oder

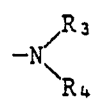

bedeuten, worin $R_3$ und $R_4$ für $C_1$-$C_3$-Alkyl stehen
sowie Salze dieser Verbindungen.

3. Mercapto-bis-(1,3,4-oxa- und -thiadiazole) gemäss Anspruch 2, worin
$X_1$ und $X_2$ voneinander unabhängig Sauerstoff oder Schwefel,
$R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,

$R_2$ Wasserstoff, $C_1$-$C_5$-Alkyl oder

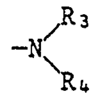

bedeuten, worin $R_3$ und $R_4$ für $C_1$-$C_3$-Alkyl stehen sowie Salze dieser Verbindungen.

4. 2-Mercapto-5-(1,3,4-oxadiazol-5-yl)-1,3,4-oxadiazole der Formel Ia

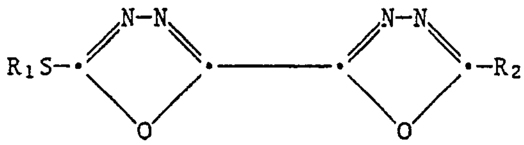

(Ia),

gemäss Anspruch 3, worin
$R_1$ Methyl, Difluormethyl, Trifluormethyl, Cyanomethyl, Allyl, Propargyl oder 3,4,4-Trifluor-3-buten-1-yl und
$R_2$ Wasserstoff, Methyl oder Dimethylamino bedeuten.

5. 2-Mercapto-5-(1,3,4-oxadiazol-5-yl)-1,3,4-oxadiazole gemäss Anspruch 4, worin
$R_1$ Difluormethyl oder 3,4,4-Trifluor-3-buten-1-yl bedeutet.

6. Das 2-Difluormethylthio-5-(2-methyl-1,3,4-oxadiazol-5-yl)-1,3,4-oxadiazol gemäss Anspruch 5.

7. Das 2-(3,4,4-Trifluor-3-buten-1-ylthio)-5-(2-methyl-1,3,4-oxadiazol-5-yl)-1,3,4-oxadiazol gemäss Anspruch 5.

8. 2-Mercapto-5-(1,3,4-thiadiazol-5-yl)-1,3,4-oxadiazole der Formel Ib

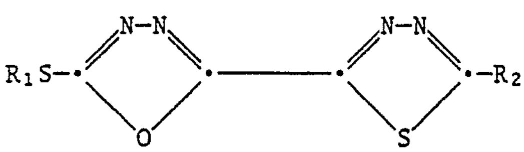

(Ib),

gemäss Anspruch 3, worin
$R_1$ Methyl, Difluormethyl, Trifluormethyl, Cyanomethyl, Allyl, Propargyl oder 3,4,4-Trifluor-3-buten-1-yl und
$R_2$ Wasserstoff, Methyl oder Dimethylamino bedeuten.

9. Das 2-Difluormethylthio-5-(2-methyl-1,3,4-oxadiazol-5-yl)-1,3,4-thiadiazol gemäss Anspruch 8.

10. Das 2-(3,4,4-Trifluor-3-buten-1-ylthio)-5-(2-methyl-1,3,4-oxadiazol-5-yl)-1,3,4-thiadiazol gemäss Anspruch 8.

11. 2-Mercapto-5-(1,3,4-oxadiazol-5-yl)-1,3,4-thiadiazole der Formel Ic

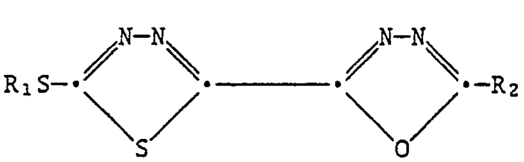

(Ic),

gemäss Anspruch 3, worin
$R_1$ Methyl, Difluormethyl, Trifluormethyl, Cyanomethyl, Allyl, Propargyl oder 3,4,4-Trifluor-3-buten-1-yl und
$R_2$ Wasserstoff, Methyl oder Dimethylamino bedeuten.

12. 2-Mercapto-5-(1,3,4-thiadiazol-5-yl)-1,3,4-thiadiazole der Formel Id

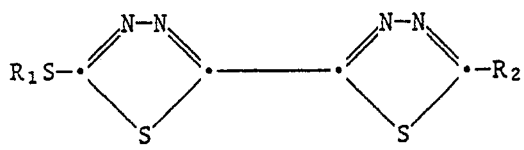

(Id),

gemäss Anspruch 3, worin
$R_1$ Methyl, Difluormethyl, Trifluormethyl, Cyanomethyl, Allyl, Propargyl oder 3,4,4-Trifluor-3-buten-1-yl und $R_2$ Wasserstoff, Methyl oder Dimethylamino bedeuten.

13. Das 2-(3,4,4-Trifluor-3-buten-1-ylthio)-5-(2-methylthio-1,3,4-thiadiazol-5-yl)-1,3,4-thiadiazol gemäss Anspruch 2.

14. Das 2-(3,4,4-Trifluor-3-buten-1-ylthio)-5-(2-methylthio-1,3,4-thiadiazol-5-yl)-1,3,4-oxadiazol gemäss Anspruch 2.

15. Das 2-(Difluormethylthio)-5-(2-methyl-1,3,4-oxadiazol-5-yl)-1,3,4-thiadiazol gemäss Anspruch 11.

16. Das 2-(3,4,4-Trifluor-3-buten-1-ylthio)-5-(2-methyl-1,3,4-oxadiazol-5-yl)-1,3,4-thiadiazol gemäss Anspruch 11.

17. Verbindungen gemäss Anspruch 1, worin $R_1$ Difluormethylthio oder 3,4,4-Trifluor-3-buten-1-yl und $R_2$ Difluormethylthio, 3,4,4-Trifluor-3-buten-1-yl, oder den Thiolrest bedeuten.

18. Das 2-(3,4,4-Trifluor-3-buten-1-ylthio)-5-(2-mercapto-1,3,4-oxadiazol-5-yl)-1,3,4-thiadiazol gemäss Anspruch 11.

19. Das 2-(3,4,4-Trifluor-3-buten-1-ylthio)-5-[2-(3,4,4-trifluor-3-buten-1-ylthio)-1,3,4-oxadiazol-5-yl]-1,3,4-thiadiazol gemäss Anspruch 11.

20. Das 2-(3,4,4-Trifluor-3-buten-1-ylthio)-5-(2-phenyl-1,3,4-oxadiazol-5-yl)-1,3,4-thiadiazol gemäss Anspruch 11.

21. Das 2-(3,4,4-Trifluor-3-buten-1-ylthio)-5-(2-phenyl-1,3,4-oxadiazol-5-yl)-1,3,4-oxadiazol gemäss Anspruch 11.

22. Das 2-(3,4,4-Trifluor-3-buten-1-ylthio)-5-(2-difluormethylthio-1,3,4-oxadiazol-5-yl)-1,3,4-thiadiazol gemäss Anspruch 11.

23. Das 2-(3,4,4-Trifluor-3-buten-1-ylthio)-5-[2-(3,4,4-trifluor-3-buten-1-ylthio)-1,3,4-oxadiazol-5-yl]-1,3,4-oxadiazol gemäss Anspruch 11.

24. Verfahren zur Herstellung der Verbindungen der Formel I gemäss Anspruch 1, dadurch gekennzeichnet, dass man

a) in einer Kondensationsreaktion eine Verbindung der Formel II

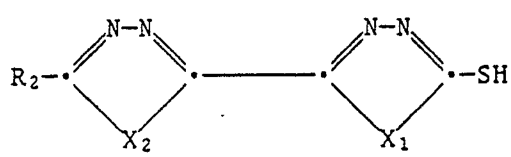

(II),

oder eine Verbindung der Formel III

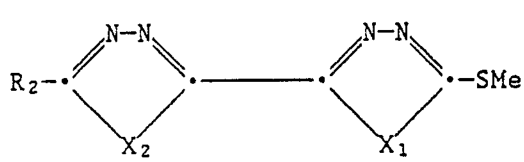

(III),

mit einer Verbindung der Formel IV
Hal-$R_1$ (IV)
in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

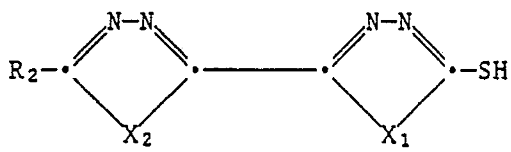

(II),

mit einer Verbindung der Formel V

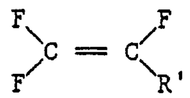

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ie

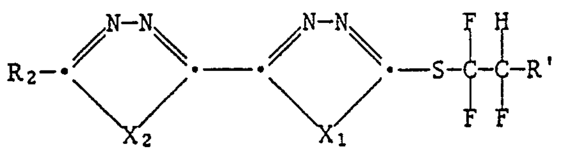

(Ie)

oder zu einer Verbindung der Formel If

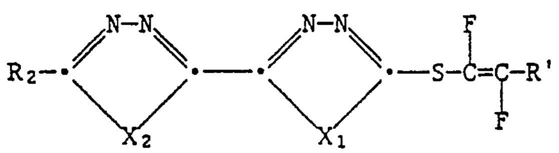

(If)

führt, wobei in den vorstehend genannten Formeln II, III, Ie, If, IV und V Me für ein Alkalimetall oder für Ammonium steht, Hal Halogen, vorzugsweise Chlor, Brom oder Jod bedeutet, und R′ Fluor oder Trifluormethyl darstellt.

25. Verfahren gemäss Anspruch 24, dadurch gekennzeichnet, dass

$R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,

$R_2$ Wasserstoff, $C_1$-$C_5$-Alkyl, unsubstituiertes oder durch Halogen substituiertes $C_1$-$C_3$-Alkoxy, unsubstituiertes oder durch Halogen substituiertes $C_1$-$C_3$-Alkylthio oder

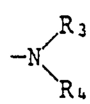

bedeuten, worin $R_3$ und $R_4$ für $C_1$-$C_3$-Alkyl stehen.

26. Verfahren gemäss Anspruch 25, dadurch gekennzeichnet, dass

$R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,

$R_2$ Wasserstoff, $C_1$-$C_5$-Alkyl oder

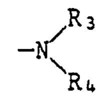

bedeuten, worin $R_3$ und $R_4$ für $C_1$-$C_3$-Alkyl stehen.

27. Verbindungen der Formel IIa

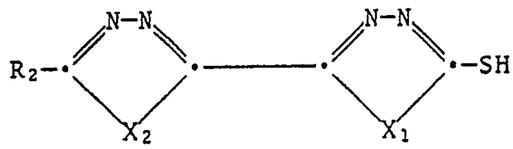

(IIa),

worin $R_2$, $X_1$ und $X_2$ die unter Formel I angegebene Bedeutungen haben, mit der Massgabe, dass $R_2$ eine von Wasserstoff verschiedene Bedeutung hat, wenn $X_1$ und $X_2$ Schwefel bedeuten.

28. Verbindungen der Formel IIa, worin $R_2$, $X_1$ und $X_2$ die unter Formel I angegebene Bedeutung haben, mit der Massgabe, dass $X_1$ und $X_2$ nicht gleichzeitig Schwefel bedeuten.

29. Schädlingsbekämpfungsmittel zur Bekämpfung oder Verhütung des Befalls von Pflanzen durch Nematoden und Pilze, dadurch gekennzeichnet, dass es als aktive Komponente mindestens eine Verbindung der Formel I, gemäss Anspruch 1 enthält.

30. Mittel gemäss Anspruch 29, dadurch gekennzeichnet, dass es als aktive Komponente mindestens eine Verbindung der Formel I gemäss der Ansprüche 2 bis 14 enthält.

31. Mittel gemäss Anspruch 29, dadurch gekennzeichnet, dass es als aktive Komponente mindestens eine Verbindung der Formel I gemäss der Ansprüche 3 bis 12 enthält.

32. Mittel gemäss Anspruch 29, dadurch gekennzeichnet, dass es als aktive Komponente 2-Difluormethylthio-5-(2-methyl-1,3,4-oxadiazol-5-yl)-1,3,4-oxadiazol enthält.

33. Mittel gemäss Anspruch 29, dadurch gekennzeichnet, dass es als aktive Komponente 2-(3,4,4-Trifluor-3-buten-1-ylthio)-5-(2-methyl-1,3,4-oxadiazol-5-yl)-1,3,4-oxadiazol enthält.

34. Mittel gemäss Anspruch 29, dadurch gekennzeichnet, dass es als aktive Komponente 2-Difluormethylthio-5-(2-methyl-1,3,4-oxadiazol-5-yl)-1,3,4-thiadiazol enthält.

35. Mittel gemäss Anspruch 29, dadurch gekennzeichnet, dass es als aktive Komponente 2-(3,4,4-Trifluor-3-buten-1-ylthio)-5-(2-methyl-1,3,4-oxadiazol-5-yl)-1,3,4-thiadiazol enthält.

36. Mittel nach einem der Ansprüche 29 bis 35, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

37. Mittel nach Anspruch 36, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

38. Verfahren zur Herstellung eines agrochemischen Mittels, dadurch gekennzeichnet, dass mindestens eine Verbindung der Formel I gemäss Anspruch 1 mit geeigneten festen oder flüssigen Zusatzstoffen und Tensiden innig vermischt.

39. Verfahren zur Bekämpfung oder Verhütung eines Befalls von Kulturpflanzen durch Nematoden und Pilze, dadurch gekennzeichnet, dass man eine Verbindung der Formel I gemäss Anspruch 1 auf die Pflanze oder deren Standort appliziert.

40. Verfahren gemäss Anspruch 39, von Verbindungen der Formel I gemäss einem der Ansprüche 2 bis 14.

41. Verfahren gemäss Anspruch 39, von Verbindungen der Formel I gemäss einem der Ansprüche 3 bis 12.

42. Verfahren gemäss Anspruch 40, dadurch gekennzeichnet, dass es sich bei den Nematoden um pflanzenparasitäre Arten handelt.

43. Verfahren gemäss Anspruch 42 gegen Nematoden der Gattung Meloidogyne, Heterodera oder Globodera.

44. Verfahren gemäss Anspruch 39 gegen Pilze der Gattungen Pythium, Rhizoctonia, Fungi imperfecti, Basidomyceten, Ascomyceten und Oomyceten.

Patentansprüche für folgenden Vertragsstaat: ES

1. Verfahren zur Herstellung von Mercapto-bis-(1,3,4-oxa- und -thiadiazole)n der Formel I

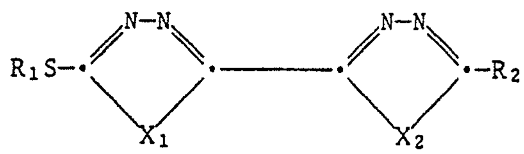
(I),

worin
$X_1$ und $X_2$ voneinander unabhängig Sauerstoff oder Schwefel,
$R_1$ Wasserstoff, unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,
$R_2$ Wasserstoff, $C_1$-$C_5$-Alkyl, unsubstituiertes oder durch Halogen substituiertes $C_1$-$C_3$-Alkoxy, unsubstituiertes oder durch Halogen substituiertes $C_1$-$C_3$-Alkylthio, unsubstituiertes oder durch Halogen substituiertes $C_3$-$C_7$-Alkenylthio, unsubstituiertes oder durch Halogen substituiertes $C_3$-$C_7$-Alkinylthio, -SH, unsubstituiertes oder durch Halogen substituiertes Phenyl oder

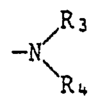

bedeuten, worin $R_3$ und $R_4$ für $C_1$-$C_3$-Alkyl stehen, dadurch gekennzeichnet, dass man
a) in einer Kondensationsreaktion eine Verbindung der Formel II

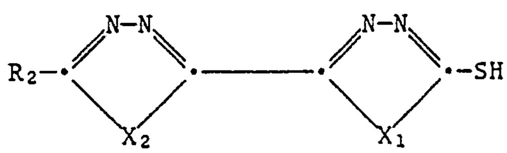
(II),

oder eine Verbindung der Formel III

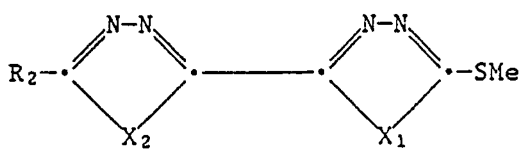
(III),

mit einer Verbindung der Formel IV
Hal-$R_1$ (IV)
in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder
b) in einer Anlagerungsreaktion eine Verbindung der Formel II

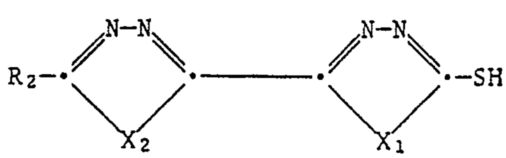
(II),

mit einer Verbindung der Formel V

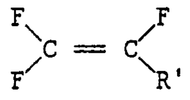 (V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ie

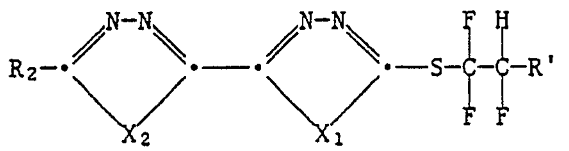 (Ie)

oder zu einer Verbindung der Formel If

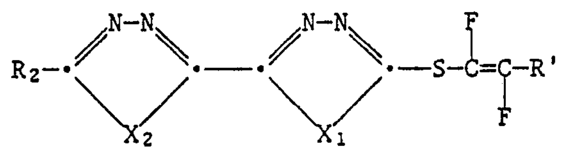 (If)

führt, wobei in den vorstehend genannten Formeln II, III, Ie, If, IV und V Me für ein Alkalimetall oder für Ammonium steht, Hal Halogen, vorzugsweise Chlor, Brom oder Jod bedeutet, und R′ Fluor oder Trifluormethyl darstellt.

2. Verfahren gemäss Anspruch 1, dadurch gekennzeichnet, dass

$R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,

$R_2$ Wasserstoff, $C_1$-$C_5$-Alkyl, unsubstituiertes oder durch Halogen substituiertes $C_1$-$C_3$-Alkoxy, unsubstituiertes oder durch Halogen substituiertes $C_1$-$C_3$-Alkylthio oder

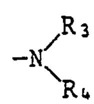

bedeuten, worin $R_3$ und $R_4$ für $C_1$-$C_3$-Alkyl stehen.

3. Verfahren gemäss Anspruch 2, dadurch gekennzeichnet, dass

$R_1$ unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl,

$R_2$ Wasserstoff, $C_1$-$C_5$-Alkyl oder

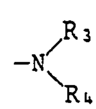

bedeuten, worin $R_3$ und $R_4$ für $C_1$-$C_3$-Alkyl stehen.

4. Verfahren gemäss Anspruch 3 zur Herstellung von 2-Mercapto-5-(1,3,4-oxadiazol-5-yl)-1,3,4-oxadiazolen der Formel Ia

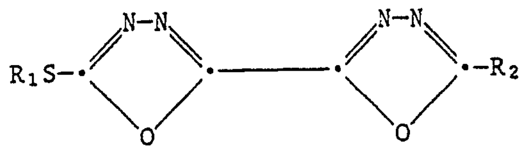

(Ia),

worin
$R_1$ Methyl, Difluormethyl, Trifluormethyl, Cyanomethyl, Allyl, Propargyl oder 3,4,4-Trifluor-3-buten-1-yl und
$R_2$ Wasserstoff, Methyl oder Dimethylamino bedeuten.

5. Verfahren gemäss Anspruch 4 zur Herstellung von 2-Mercapto-5-(1,3,4-oxadiazol-5-yl)-1,3,4-oxadiazolen, worin
$R_1$ Difluormethyl oder 3,4,4-Trifluor-3-buten-1-yl bedeutet.

6. Verfahren gemäss Anspruch 5 zur Herstellung von 2-Difluormethylthio-5-(2-methyl-1,3,4-oxadiazol-5-yl)-1,3,4-oxadiazol.

7. Verfahren gemäss Anspruch 5 zur Herstellung von 2-(3,4,4-Trifluor-3-buten-1-ylthio)-5-(2-methyl-1,3,4-oxadiazol-5-yl)-1,3,4-oxadiazol.

8. Verfahren gemäss Anspruch 3 zur Herstellung von 2-Mercapto-5-(1,3,4-thiadiazol-5-yl)-1,3,4-oxadiazolen der Formel Ib

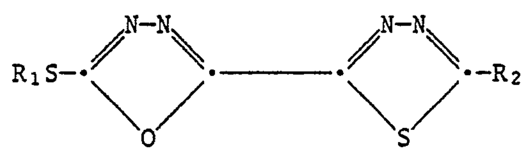

(Ib),

worin
$R_1$ Methyl, Difluormethyl, Trifluormethyl, Cyanomethyl, Allyl, Propargyl oder 3,4,4-Trifluor-3-buten-1-yl und
$R_2$ Wasserstoff, Methyl oder Dimethylamino bedeuten.

9. Verfahren gemäss Anspruch 8 zur Herstellung von 2-Difluormethylthio-5-(2-methyl-1,3,4-oxadiazol-5-yl)-1,3,4-thiadiazol.

10. Verfahren gemäss Anspruch 8 zur Herstellung von 2-(3,4,4-Trifluor-3-buten-1-ylthio)-5-(2-methyl-1,3,4-oxadiazol-5-yl)-1,3,4-thiadiazol.

11. Verfahren gemäss Anspruch 3 zur Herstellung von 2-Mercapto-5-(1,3,4-oxadiazol-5-yl)-1,3,4-thiadiazolen der Formel Ic

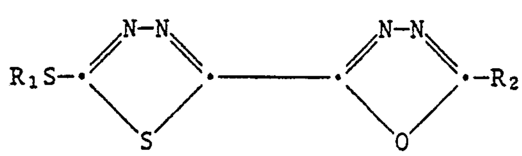

(Ic),

worin
$R_1$ Methyl, Difluormethyl, Trifluormethyl, Cyanomethyl, Allyl, Propargyl oder 3,4,4-Trifluor-3-buten-1-yl und
$R_2$ Wasserstoff, Methyl oder Dimethylamino bedeuten.

12. Verfahren gemäss Anspruch 3 zur Herstellung von 2-Mercapto-5-(1,3,4-thiadiazol-5-yl)-1,3,4-thiadiazolen der Formel Id

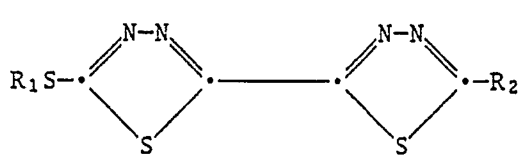

(Id),

worin

$R_1$ Methyl, Difluormethyl, Trifluormethyl, Cyanomethyl, Allyl, Propargyl oder 3,4,4-Trifluor-3-buten-1-yl und $R_2$ Wasserstoff, Methyl oder Dimethylamino bedeuten.

13. Verfahren gemäss Anspruch 2 zur Herstellung von 2-(3,4,4-Trifluor-3-buten-1-ylthio)-5-(2-methylthio-1,3,4-thiadiazol-5-yl)-1,3,4-thiadiazol.

14. Verfahren gemäss Anspruch 2 zur Herstellung von 2-(3,4,4-Trifluor-3-buten-1-ylthio)-5-(2-methylthio-1,3,4-thiadiazol-5-yl)-1,3,4-oxadiazol.

15. Verfahren gemäss Anspruch 11 zur Herstellung von 2-(Difluormethylthio)-5-(2-methyl-1,3,4-oxadiazol-5-yl)-1,3,4-thiadiazol.

16. Verfahren gemäss Anspruch 11 zur Herstellung von 2-(3,4,4-Trifluor-3-buten-1-ylthio)-5-(2-methyl-1,3,4-oxadiazol-5-yl)-1,3,4-thiadiazol.

17. Verfahren zur Herstellung von Verbindungen gemäss Anspruch 1, worin $R_1$ Difluormethylthio oder 3,4,4-Trifluor-3-buten-1-yl und $R_2$ Difluormethylthio, 3,4,4-Trifluor-3-buten-1-yl, oder den Thiolrest bedeuten.

18. Verfahren gemäss Anspruch 11 zur Herstellung von 2-(3,4,4-Trifluor-3-buten-1-ylthio)-5-(2-mercapto-1,3,4-oxadiazol-5-yl)-1,3,4-thiadiazol.

19. Verfahren gemäss Anspruch 11 zur Herstellung von 2-(3,4,4-Trifluor-3-buten-1-ylthio)-5-[2-(3,4,4-trifluor-3-buten-1-ylthio)-1,3,4-oxadiazol-5-yl]-1,3,4-thiadiazol.

20. Verfahren gemäss Anspruch 11 zur Herstellung von 2-(3,4,4-Trifluor-3-buten-1-ylthio)-5-(2-phenyl-1,3,4-oxadiazol-5-yl)-1,3,4-thiadiazol.

21. Verfahren gemäss Anspruch 11 zur Herstellung von 2-(3,4,4-Trifluor-3-buten-1-ylthio)-5-(2-phenyl-1,3,4-oxadiazol-5-yl)-1,3,4-oxadiazol.

22. Verfahren gemäss Anspruch 11 zur Herstellung von 2-(3,4,4-Trifluor-3-buten-1-ylthio)-5-(2-difluormethylthio-1,3,4-oxadiazol-5-yl)-1,3,4-thiadiazol.

23. Verfahren gemäss Anspruch 11 zur Herstellung von 2-(3,4,4-Trifluor-3-buten-1-ylthio)-5-[2-(3,4,4-trifluor-3-buten-1-ylthio)-1,3,4-oxadiazol-5-yl]-1,3,4-oxadiazol.

Europäisches Patentamt

# EUROPÄISCHER RECHERCHENBERICHT

Nummer der Anmeldung

EP 89 81 0688

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 217 747 (CIBA-GEIGY)<br>* Seite 18, Anspruch 1; Seite 20, Anspruch 12 *<br>--- | 1,29 | C 07 D 413/04<br>C 07 D 417/04<br>A 01 N 43/82 //<br>(C 07 D 413/04<br>C 07 D 271:00<br>C 07 D 271:00 )<br>(C 07 D 417/04<br>C 07 D 285:00<br>C 07 D 285:00 )<br>(C 07 D 417/04<br>C 07 D 285:00<br>C 07 D 271:00 ) |
| A | EP-A-0 263 066 (CIBA-GEIGY)<br>* Seite 18, Anspruch 1; Seite 20, Anspruch 11 *<br>--- | 1,29 | |
| A,P | EP-A-0 285 565 (CIBA-GEIGY)<br>* Seite 19, Anspruch 1; Seite 20, Anspruch 30 *<br>--- | 1,29 | |
| A,P | EP-A-0 290 379 (CIBA-GEIGY)<br>* Seite 12, Anspruch 1; Seite 14, Anspruch 9 *<br>----- | 1,29 | |
| | | | RECHERCHIERTE SACHGEBIETE (Int. Cl.5) |
| | | | C 07 D 413/00<br>C 07 D 417/00<br>A 01 N 43/82 |

Der vorliegende Recherchenbericht wurde für alle Patentansprüche erstellt

| Recherchenort | Abschlußdatum der Recherche | Prüfer |
|---|---|---|
| BERLIN | 18-12-1989 | KYRIAKAKOU G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..................................................
& : Mitglied der gleichen Patentfamilie, übereinstimmendes Dokument

EPO FORM 1503 03.82 (P0403)